(19) Europäisches Patentamt
European Patent Office
Office européen des brevets

(11) Veröffentlichungsnummer: **0 091 990**
**B1**

(12) # EUROPÄISCHE PATENTSCHRIFT

(45) Veröffentlichungstag der Patentschrift: **18.03.87**

(51) Int. Cl.⁴: **A 61 K 6/06**, A 61 K 6/08

(21) Anmeldenummer: **82111183.8**

(22) Anmeldetag: **02.12.82**

(54) **Dentales Restorationsmaterial.**

(30) Priorität: **15.04.82 US 368743**

(43) Veröffentlichungstag der Anmeldung: **26.10.83 Patentblatt 83/43**

(45) Bekanntmachung des Hinweises auf die Patenterteilung: **18.03.87 Patentblatt 87/12**

(84) Benannte Vertragsstaaten: **AT BE CH DE FR GB IT LI LU NL SE**

(56) Entgegenhaltungen:
**FR-A-2 370 467**
**GB-A-1 544 776**

(73) Patentinhaber: **Blendax- Werke R. Schneider GmbH & Co., Rheinallee 88, D-6500 Mainz (DE)**

(72) Erfinder: **Orlowski, Jan A., Dr., 1304 Rubio Street, Altadena California, 91001 (US)**

**EP 0 091 990 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft ein neues dentales Restorationsmaterial, das ein spezielles Fullstoffgemisch enthält.

Dentale Füllungsmaterialien auf der Basis polymerisierbarer Verbindungen, sogenannte "Composites", enthalten neben einem oder mehreren polymerisierbaren Monomeren, Aktivatoren, ggf. Polymerisationskatalysatoren und sonstigen Bestandteilen obligatorisch einen mineralischen Füllstoff.

Dieser Füllstoff ist nach Art und Menge bestimmend für die physikalischen Eigenschaften der durch das Composite hergestellten Füllung. Je höher der Füllstoffanteil und dessen Teilchengrößen, desto besser für die physikalischen Eigenschaften, desto schlechter jedoch in der Regel die Polierbarkeit.

Man hat deshalb versucht, die Polierbarkeit derartiger Materialien durch den Einsatz von Fullstoffen mit niederen Teilchengrößen zwischen etwa 10 und 300 nm zu verbessern; dies geht jedoch auf Kosten der mechanischen Eigenschaften.

Diese sogenannten "Mikrofiller" finden insbesondere überwiegend oder nahezu ausschließlich Verwendung bei der Herstellung von sogenannten lichthärtenden Composites, d.h. dentalen Restorationsmaterialien, die in einer Phase vorliegen und Füllstoffe, polymerisierbare Verbindungen und einen unter Lichteinfluß Radikale bildenden Polymerisationsinitiator enthalten.

Dies beruht insbesondere darauf, daß diese Materialien eine bestimmte Aushärtungstiefe aufweisen müssen, die mit den meisten Füllstoffen höherer Teilchengrößen, den sogenannten "Makrofillern", nicht erreicht wird.

Andere Makrofiller, die diesen Nachteil nicht aufweisen, verursachen Verfarbungen bei der Polymerisation (= Aushärtung) der Füllung. Dies gilt insbesondere fur die verschiedenen Glassorten, die bei alleiniger Benutzung eine grünliche oder gräuliche Verfärbung während der Aushärtung der Füllung ergeben und darüber hinaus auch nicht polierbar sind.

Es bestand daher ein Bedürfnis, dentale Restorationsmaterialien, insbesondere lichthärtende, zu entwicklen, die diese Nachteile nicht aufweisen, sondern auch ohne Polymerisationskatalysatoren unter Lichteinfluß gut aushärtbar sind, d.h. eine befriedigende Aushartungstiefe aufweisen, keine Verfärbung ergeben, jedoch gute physikalische Eigenschaften aufweisen, insbesondere im Hinblick auf eine verringerte Wasserabsorption, Schrumpfung, einen auf Null hin tendierenden thermischen Ausdehnungskoeffizienten und verbesserte mechanische Eigenschaften, insbesondere hinsichtlich Härte und diametraler Zugfestigkeit.

Darüber hinaus ist es erwunscht, wenigstens einen gewissen Grad an Polierbarkeit zu erreichen.

Es wurde nun gefunden, daß sich ein dentales Restorationsmaterial mit den beschriebenen Eigenschaften herstellen läßt, wenn man in einem solchen Mittel als anorganisches Füllungsmaterial in einer Menge von 60 bis 90 Gew.-% der Gesamtzusammensetzung ein Gemisch aus mindestens einem gegebenenfalls silanisierten amorphen Füllstoff, in einer Menge von 50 bis 90, insbesondere 50 bis 75 Gew.-% des Füllstoffgemisches, mindestens einen gegebenenfalls silanisierten kristallinen Fullstoff in einer Menge von 10 bis 50, insbesondere 25 bis 50 Gew.-% des Fullstoffgemisches mit jeweils mittleren Teilchendurchmessern zwischen etwa 0,3 und etwa 40 µm, vorzugsweise 0,3 und 20 µm, verwendet, wobei dieses Gemisch zur Verbesserung der Polierfähigkeit noch bis zu etwa 10 Gew.-% eines gegebenenfalls silanisierten Mikrofüllstoffs, insbesondere feinverteiltem Siliciumdioxid mit einem mittleren Teilchendurchmesser von weniger als 0,2 µm, enthalten kann.

Geeignete amorphe Füllungsmaterialien sind insbesondere verschiedene Glassorten wie Lithiumaluminiumsilikat-Glas, pulverisierter Quarz, Borsilikat-Glas, Bariumaluminiumsilikat, Bariumaluminiumboratsilikat odr Glaskeramikfüllstoffe mit Teilchengrößen zwischen etwa 0,5 und etwa 40, vorzugsweise zwischen 1 und 20 µm. Diese amorphen Füllstoffe als Komponente des erfindungsgemäß zum Einsatz gelangenden Fullstoffgemisches können röntgendurchlassig oder röntgenundurchlassig sein. Eine Zusammenfassung solcher geeigneter Füllungsmaterialien findet sich beispielsweise bei R.L. Bowen, Journal of Dental Research, Vol. 58/5 (Mai 1979), S. 1493-1501, insbesondere S. 1495-1498.

Geeignete röntgenopake Fullstoffe sind insbesondere in den US-A-3 801 344, 3 808 170 und 3 975 203 sowie der DE-A-2 347 591 beschrieben.

Als geeigneter kristalliner Bestandteil des erfindungsgemäßen Füllstoffgemisches hat sich insbesondere kristallines Aluminiumsilikat, beispielsweise aus gefalltem Natronfeldspat, erwiesen. Weitere geeignete kristalline Füllstoffkomponenten sind beispielsweise Lithiumaluminiumsilikat wie Beta-Eucryptit, synthetisches oder natürliches Calciumsilikat, mit einer mittleren Teilchengröße zwischen etwa 0,3 und 40 µm, vorzugsweise etwa 0,3 bzw. 0,5 und 20 bzw. 10 µm.

Aus der GB-A-1 544 776 sind bereits dentale Restorationsmaterialien bekannt (vgl. insbes. Beispiel 3), die als Fullstoff ein Gemisch aus Quarz und kolloidalem Siliciumdioxid enthalten können.

Einen Hinweis auf die erfindungsgemäße Kombination aus kristallinen und amorphen Füllstoffen im Teilchengrößenbereich zwischen 0,3 und 40 µm läßt sich daraus nicht ableiten.

Um die Einarbeitbarkeit des erfindungsgemäßen Füllstoffgemisches in die Zusammensetzung und die Verträglichkeit mit den organischen Bestandteilen zu verbessern, ist es zweckmäßig, diese Füllstoffe mit einem Organosilan zu silanisieren. Die Silanisierung kann mit jedem geeigneten Organosilan der allgemeinen Formel

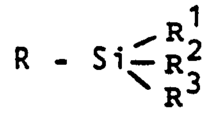

wobei R, $R^1$, $R^2$ und $R^3$ gleiche oder verschiedene organische Reste darstellen mit der Maßgabe, daß mindestens ein Rest eine OH-Gruppe oder einen in eine OH-Gruppe, beispielsweise durch Hydrolyse, überführbaren Rest, insbesondere eine Alkoxygruppe, bedeutet, erfolgen. Bevorzugte Organosilane sind (Meth)Acroylpropyldihydroxymethoxysilan, (Meth)Acroylpropylhydroxidimethoxysilan, (Meth)Acroylpropyltrimethoxysilan oder deren Gemische; jedoch sind beispielsweise auch Vinyltriethoxysilan oder Vinyltri(methoxyethoxy)silan geeignete Silanisierungsmittel.

Um die Polierbarkeit des anmeldungsgemäßen dentalen Restorationsmaterials zu verbessern, kann auch noch ein gewisser Anteil eines Mikrofillers, insbesondere eines feinteiligen Siliciumdioxids, mit einem mittleren Teilchendurchmesser unterhalb von 200 nm, vorhanden sein. Der Anteil dieses Mikrofillers sollte jedoch, um die guten physikalischen Eigenschaften des ausgehärteten Materials nicht zu beeinflussen, nicht mehr als 10 Gew.-%, vorzugsweise 4 bis 8, insbesondere etwa 6 Gew.-% des Gesamtfüllstoffanteils betragen. Auch dieser Mikrofüllstoff ist vorzugsweise silanisiert. Ein geeignetes silanisiertes Siliciumdioxid-Füllungsmaterial ist in der EP-A-0 060 911 beschrieben.

Ein besonders geeignetes Füllstoffgemisch ist ein solches, das 50 bis 75 Gew.-%. gegebenenfalls silanisiertes amorphes Lithiumaluminiumsilikat mit einer Teilchengröße zwischen etwa 1 und etwa 35 µm (im Mittel etwa 5 µm) und etwa 25 bis etwa 50 Gew.-% eines gegebenenfalls silanisierten kristallinen Aluminiumsilikats mit einer mittleren Teilchengröße zwischen etwa 0,3 und etwa 20 µm, insbesondere 0,5 bis 1 µm im Mittel, enthält, vorzugsweise im Gewichtsverhältnis 3:1.

Wie bereits ausgeführt, eignen sich die erfindungsgemäßen dentalen Restorationsmaterialien insbesondere zur Anwendung als lichthärtende Produkte, d.h., Produkte, die in einer Phase vorliegen und unter Einwirkung von Licht polymerisieren.

Solche Zusammensetzungen enthalten einen oder mehrere Photopolymerisationsinitiatoren. Als solche sind insbesondere Carbonylverbindungen wie Benzoin und dessen Derivate, insbesondere Benzoinmethyläther, Benzil und Benzilderivate, beispielsweise 4,4-Oxidibenzil oder andere Dicarbonylverbindungen, z. B. Diacetyl, 2,3-Pentandion oder Metallcarbonyle, Chinone oder deren Derivate, geeignet. Der Anteil an Photopolymerisationsinitiator beträgt 0,01 bis 5 Gew.-% der Gesamtzusammensetzung.

Diese im Licht härtbaren, d.h. photopolymerisierbaren Präparate enthalten vorzugsweise auch noch sogenannte Polymerisationsbeschleuniger. Dies sind Substanzen, die in Gegenwart von Polymerisationsinitiatoren die Polymerisationsreaktion beschleunigen. Bekannte Beschleuniger sind beispielsweise Amine wie p-Toluidin, N,N-Dimethyl-p-toluidin, N,N-Di(hydroxyethyl)-p-toluidin, Trialkylamine wie Trihexylamin, Polyamine wie N,N,N',N'-Tetraalkylalkylendiamine, Barbitursäure und Dialkylbearbitursäuren und Sulfimide, vorzugsweise in einer Menge von 0,01 bis 5 Gew.-% der Gesamtzusammensetzung. Geeignete Acceleratoren sind beispielsweise bei G.M. Brauer et. al. Journal of Dental Research, Vol. 58/No. 10 (1979), S 1994-2000 beschrieben.

Ein bevorzugtes lichthärtbares dentales Restorationsmaterial enthält 60 bis 90 Gew.-% der Gesamtzusammensetzung eines anorganischen Füllstoffgemisches, das aus 60 bis 75 Gew.-% (berechnet auf das Füllstoffgemisch) eines gegebenenfalls silanisierten amorphen Füllstoffs, insbesondere Lithiumaluminiumsilikat, mit einer Teilchengröße zwischen etwa 1 und etwa 20 µm, und 25 bis 40 Gew.-% eines gegebenenfalls silanisierten kristallinen Füllstoffs, insbesondere Aluminiumsilikat, mit einer Teilchengröße von etwa 0,5 bis etwa 1 µm besteht. Gegebenenfalls kann dieses Füllstoffgemisch noch bis zu 10, vorzugsweise bis zu 6 Gew.-% eines vorzugsweise silanisierten feinverteilten Siliciumdioxids enthalten, das einen mittleren Teilchendurchmesser von weniger als 0,2 µm aufweist.

Im Prinzip ist es auch möglich, die erfindungsgemäßen dentalen Restorationsmaterialien als zweiphasige Präparate einzusetzen, von denen die eine Phase einen Polymerisationskatalysator, beispielsweise ein Peroxid, und die andere Phase einen Beschleuniger für dieses Peroxid, beispielsweise ein organisches Amin, enthält, wobei das Zusammenbringen beider Phasen unmittelbar vor der Zahnfüllung erfolgt und die Polymerisation in der aufgebohrten, vorzugsweise mit einem Unterfütterungs- oder einem Bondingmaterial versehenen, zu füllenden Kavität eintritt.

Geeignete Peroxide, die bei der Auslösung der Polymerisation unter Radikalbildung zerfallen, sind beispielsweise Arylperoxide wie Benzoylperoxid, Cumolhydroperoxid, Harnstoffperoxid, tert.-Butylhydroperoxid oder -perbenzoat und Silylperoxide, vorzugsweise in Mengen von 0,01 bis 5, insbesondere 0,5 bis 2,5 Gew.-% der Gesamtzusammensetzung.

Enthält die eine Phase des zweiphasigen Mittels einen Polymerisationsinitiator, so wird der anderen Phase zweckmäßigerweise ein Beschleuniger des oben beschriebenen Typs, vorzugsweise ein Amin oder Barbitursäure oder deren Derivate, beispielsweise eine Dialkylbarbitursäure, zugesetzt.

Als polymerisierbare Monomere in den erfindungsgemäßen dentalen Restorationsmaterialien sind polymerisierbare (Meth)Acrylverbindungen einsetzbar. Hier seien insbesondere die bekannten Reaktionsprodukte aus Bisphenolen, insbesondere Bisphenol A, und Glycidylmethacrylat, unter der Abkürzung Bis-GMA bekannt, die verschiedenen Alkandioldimethacrylate wie 1,6-Hexan-diolmethacrylat, 1,4-Butandioldimethacrylat, Tri- oder Tetraethylenglykoldimethacrylat, Bis (2-methacroylpropyl)-phthalat, -isophthalat oder -terephthalat, Trimethylolpropandi- und trimethacrylat, sowie insbesondere die Reaktionsprodukte aus Diisocyanaten und Hydroxyalkylmethacrylaten, wie sie beispielsweise in der DE-A- No. 2 312 559 beschrieben sind, Addukte aus (Di)Isocyanaten und 2,2-Propan-bis-[3-(4-phenoxy)-1,2-hydroxypropan]-1-methacrylat nach der US-A- No. 3 629 187 sowie insbesondere die Addukte aus Isocyanaten und Methacroylalkylethern, -alkoxybenzolen bzw. -alkoxycycloalkanen, wie sie in der EP-A-44 352 beschrieben sind, genannt.

Selbstverständlich können auch Gemische aus geeigneten Monomeren verwendet werden.

Es ist schließlich zweckmäßig, dentalen Füllungsmaterialien auf Kunststoffbasis UV-Stabilisatoren zuzusetzen, um das Nachdunkeln während des Alterns der Füllungen zu vermeiden. Ein besonders geeigneter UV-Stabilisator ist 2-Hydroxy-4-methoxybenzophenon. Ein weiteres bevorzugtes Material ist 2-(2'-Hydroxy-5'-methylphenyl)benzotriazol; jedoch ist prinzipiell jedes physiologisch inerte UV-absorbierende Agens für diesen Zweck geeignet.

So seien beispielhaft noch Hydrochinon, p-Benzochinon, p-Butylhydroxytoluol u.a. genannt. Die letztere Verbindung kann beispielsweise auch als Antioxidans in der Füllung wirken.

Eine Übersicht über die in dentalen Füllungsmaterialien üblicherweise zum Einsatz gelangenden Substanzen findet sich in dem bereits erwähnten Artikel von R.L. Bowen im Journal of Dental Research, Vol. 58/5 (Mai 1979), S. 1493 bis 1503, sowie der daran angeschlossenen Ergänzungen von J.F. Lann, S. 1504 bis 1506.

Zur Einstellung eines möglichst naturgetreuen Eindrucks der gefüllten Zahnflächen enthalten Composite-Materialien erforderlichenfalls auch einen geringen Anteil an Farbstoffen oder Pigmenten.

Die nachfolgenden Beispiele dienen der Erläuterung der Erfindung.

**Mischung A**

| | |
|---|---|
| Reaktionsprodukt aus Glycidylmethacrylat und Bisphenol A (Bis-GMA) | 80 Gew.-% |
| 1,6-Hexandioldimethacrylat | 20 Gew.-% |
| Benzil | 0,75 } Gew.-Teile/100 Gew.-Teile. Misch.A |
| Diethylaminoethylmethacrylat | 0,75 } |
| 2-(2'-Hydroxy-5'-methylphenyl)benzotriazol | 0,4 } |

**Mischung B**

| | |
|---|---|
| Bis-GMA | 70 Gew.-% |
| 1,6-Hexandioldimethacrylat | 18 Gew.-% |
| Trimethylolpropantrimethacrylat | 12 Gew.-% |
| Benzil | 0,5 } Gew.-Teile/100 Gew.-Teile. Misch.B |
| Diethylaminoethylmethacrylat (DEAEM) | 0,4 } |

**Mischung C**

| | | |
|---|---|---|
| Bis-GMA | 66,5 | Gew.-% |
| 1,6-Hexandioldimethacrylat | 25 | Gew.-% |
| Ethoxyliertes Bisphenol A-Dimethacrylat (EBA) | 3,5 | Gew.-% |
| Methylen-4,4',N,N'-biscyclohexylcarbamat des 3-Methacroyl-2-hydroxypropoxytoluols | 1,5 | Gew.-% |
| Triethylenglykoldimethacrylat | 3,5 | Gew.-% |
| Benzil | 0,66 | Gew.-Teile/100 Gew.-Teile Mischung C |
| DEAEM | 0,82 | |
| 2-(2'-Hydroxy-5'-methylphenyl)benzotriazol | 0,13 | |

**BEISPIELE**

Die Beispiele I bis III sind nichterfindungsgemäße Zusammensetzungen.

**Beispiel I**

Mischung A 100 Gew.-Teile
Amorphes Quarzpulver (Teilchengröße ≈ 5 µm) 330 Gew.-Teile
Eigenschaften des gehärteten Materials:
Härtungstiefe: 3,2 mm
Polierbarkeit: Keine.
Aussehen: Unbefriedigend; zu durchsichtig mit Grünstich.

**Beispiel II**

Mischung B 100 Gew.-Teile
Silanisiertes amorphes Quarzpulver (Teilchengröße ≈ 5 µm) 125 Gew.-Teile
Silanisiertes-Siliciumdioxid (Teilchengröße ≈ 20,5 nm) 45 Gew.-Teile
Eigenschaften des gehärteten Materials:
Härtungstiefe: 2,5 mm

Polierbarkeit: Gut.
Aussehen: Mäßig. Das gehärtete Material hat einen Gelbstich.

**Beispiel III**

Mischung C 100 Gew.-Teile
Lithiumaluminiumsilikat-Glas, Teilchengröße 1 bis 35 µm, im Mittel 4 µm 500 Gew.-Teile
Eigenschaften des gehärteten Materials:
Härtungstiefe: 5,0 mm
Polierbarkeit: Keine.
Aussehen: Zu durchsichtig, mit Graustich.
Die Beispiele 1 bis 7 sind erfindungsgemäße Zusammensetzungen.

**Beispiel 1**

Mischung A 100 Gew.-Teile
Silanisiertes amorphes Quarzpulver (Mittlere Teilchengröße unter 5 µm) 250 Gew.-Teile
Kristallines Lithiumaluminiumsilikat (Teilchendurchmesser 1 bis 35 µm, im Mittel 4 µm) 100 Gew.-Teile
Eigenschaften des gehärteten Materials:
Härtungstiefe: 3,7 mm
Polierbarkeit: Gut.
Aussehen: Sehr gut; die optischen Eigenschaften des gehärteten Materials entsprechen denen der Zahnstruktur ohne unerwünschte Verfärbung.

**Beispiel 2**

Mischung B 100 Gew.-Teile
Silanisiertes Borsilikat-Glas (Teilchengröße 0,5 bis 35 µm, im Mittel 3 µm) 450 Gew.-Teile
Silanisiertes kristallines Aluminiumsilikat (Mittlere Teilchengröße 0,5 bis 1 µm) 150 Gew.-Teile
Eigenschaften des gehärteten Materials:
Härtungstiefe: 4,0 mm
Polierbarkeit: Gut.
Aussehen: Sehr gut. Die optischen Eigenschaften des gehärteten Materials entsprechen denen der Zahnstruktur ohne unerwünschte Verfärbung.

**Beispiel 3**

Mischung C 100 Gew.-Teile
Lithiumaluminiumsilikat-Glas (Teilchengröße 1 bis 35 µm, im Mittel 4 µm) 450 Gew.-Teile
Silanisiertes kristallines Aluminiumsilikat (Mittlere Teilchengröße 0,5 bis 1,5 µm) 150 Gew.-Teile
Eigenschaften des gehärteten Materials:
Härtungstiefe: 4,2 mm
Polierbarkeit: Gut.
Aussehen: Sehr gut. Die optischen Eigenschaften des gehärteten Materials entsprechen denen der Zahnstruktur ohne unerwünschte Verfärbung.

**Beispiel 4**

Mischung C 100 Gew.-Teile
Silanisiertes Borsilikat-Glas (Teilchengröße 0,5 bis 35 µm, im Mittel 3 µm) 450 Gew.-Teile
Kristallines Lithiumaluminiumsilikat (Beta-Eucryptit) (Mittlerer Teilchendurchmesser 1 µm) 100 Gew.-Teile
Eigenschaften des gehärteten Materials:
Härtungstiefe: 4,4 mm
Polierbarkeit: Gut.
Aussehen: Sehr gut. Die optischen Eigenschaften des gehärteten Materials entsprechen denen der

Zahnstruktur ohne unerwünschte Verfärbung.

**Beispiel 5**

Mischung B 100 Gew.-Teile
Silanisiertes amorphes Quarzpulver (Teilchengröße < 5 µm) 150 Gew.-Teile
Silanisiertes kristallines Lithiumaluminiumsilikat (Beta-Eucryptit) (Mittlerer Teilchendurchmesser 1 µm) 150 Gew.-Teile
Silanisiertes gefälltes Siliciumdioxid (Teilchengröße 30 bis 150 nm) 20 Gew.-Teile
Eigenschaften des gehärteten Materials:
Härtungstiefe: 3,6 mm
Polierbarkeit: Ausgezeichnet.
Aussehen: Gut; keine wahrnehmbare Verfärbung.

**Beispiel 6**

Mischung C 100 Gew.-Teile
Lithiumaluminiumsilikat-Glas (Teilchengröße 1 bis 35 µm, im Mittel 4 µm) 250 Gew.-Teile
Kristallines Aluminiumsilikat (Mittlerer Teilchendurchmesser 0,5 bis 1 µm) 250 Gew.-Teile
Eigenschaften des gehärteten Materials:
Härtungstiefe: 3,1 mm
Polierbarkeit: Gut.
Aussehen: Befriedigend; jedoch geringfügig opaker als die natürliche Zahnstruktur.

**Beispiel 7**

Mischung C 100 Gew.-Teile
Lithiumaluminiumsilikat-Glas (Teilchengröße 1 bis 35 µm, im Mittel 4 µm) 500 Gew.-Teile
Kristallines Lithiumaluminiumsilikat (Beta-Eucryptit) (Mittlerer Teilchendurchmesser 1 µm) 60 Gew.-Teile
Eigenschaften des gehärteten Materials:
Härtungstiefe: 5,5 mm
Polierbarkeit: Befriedigend,
Aussehen: Befriedigend; jedoch etwas transparent.

Die Härtungstiefe wurde jeweils mit einer Härtungslampe ("Translux") bei 40-sekündiger Belichtungszeit ermittelt. Die lichtinduzierte Verfärbung wurde nach der ADA-Spezifikation Nr. 27 bestimmt.

Die Ergebnisse zeigen die überlegene Wirkung der erfindungsgemäßen Zusammensetzungen.

**Patentansprüche**

1. Dentales Restorationsmaterial, enthaltend eine oder mehrere polymerisierbare (Meth)Acrylverbindungen, Polymerisationsbeschleuniger und/oder Polymerisationsinitiatoren und gegebenenfalls weitere in solchen Mitteln an sich übliche Zusatze sowie 60 bis 90 Gew.-% eines anorganisches Füllstoffgemisches, dadurch gekennzeichnet, daß es als Füllstoffgemisch ein Gemisch aus
a) 50 bis 90 Gew.-% mindestens einem gegebenenfalls silanisierten amorphen Füllstoff
und
b) 10 bis 50 Gew.-% mindestens einem gegebenenfalls silanisierten kristallinen Füllstoff
mit jeweils einem mittleren Teilchendurchmesser zwischen etwa 0,3 und etwa 40 µm, und
c) gegebenenfalls bis zu 10 Gew.-% eines Mikrofüllstoffs mit einem mittleren Teilchendurchmesser von weniger als 0,2 µm enthalt.

2. Dentales Restorationsmaterial nach Anspruch 1, dadurch gekennzeichnet, daß es als Füllstoffgemisch ein solches aus 50 bis 75 Gew.-% gegebenenfalls silanisiertem amorphen Lithiumaluminiumsilikat und 25 bis 50 Gew.-% eines gegebenenfalls silanisierten kristallinen Aluminiumsilikats, jeweils mit einem mittleren Teilchendurchmesser zwischen etwa 0,3 und etwa 20 µm, und gegebenenfalls bis zu 10 Gew.-% eines gegebenenfalls silanisierten Siliciumdioxids mit einem mittleren Teilchendurchmesser von weniger als 0,1 µm enthält.

3. Lichthartbares dentales Restorationsmaterial, enthaltend eine oder mehrere polymerisierbare (Meth)Acrylverbindungen, Polymerisationsinitiatoren und/oder Polymerisationsbeschleuniger, gegebenenfalls

weitere in solchen Mitteln übliche Zusätze und 60 bis 90 Gew.-% der Gesamtzusammensetzung eines anorganischen Füllstoffgemisches, dadurch gekennzeichnet, daß das Füllstoffgemisch aus

a) 60 bis 75 Gew.-% eines gegebenenfalls silanisierten amorphen Füllstoffs mit einer Teilchengröße zwischen 1 und 20 µm,

b) 25 bis 40 Gew.-% eines gegebenenfalls silanisierten kristallinen Füllstoffs mit einer Teilchengröße von 0,5 bis 1 µm,

und

c) gegebenenfalls bis zu 10 Gew.-% eines gegebenenfalls silanisierten feinverteilten Siliciumdioxids mit einer Teilchengröße von weniger als 0,2 µm besteht.

## Claims

1. Dental restoring material, containing one or more polymerizable (meth)acrylic compound(s), polymerization accelerators and (or) initiators and optionally additional components usually present in these materials, and 60 to 90 % by weight of a mixture of inorganic filler compounds, characterized in that said filler mixture is composed of

a) 50 to 90 % by weight of an optionally silanized amorphous filling material, and

b) 10 to 50 % by weight of an optionally silanized crystalline filling material, having each particle diameters between about 0,3 and about 40 µm, and

c) optionally up to 10 % by weight of a microfiller with an average particle size of not more than 0,2 µm.

2. Dental restoring material according to claim 1, characterized in that the filler mixture is composed of 50 to 75 % by weight of optionally silanized amorphous lithium aluminum silicate, and 25 to 50 % by weight of an optionally silanized crystalline aluminum silicate, both having an average particle size from about 0,3 to 20 µm, and optionally up to 10 % by weight of an optionally silanized silica having an average particle size of less than 0,1 µm.

3. A light-curable dental restoring material, containing at least one polymerizable (meth)acrylic compound , a polymerization initiator, and (or) a polymerization accelerator, and optionally additional components usually present in these materials, and 60 to 90 % by weight of the total composition of a mixture of inorganic filling materials being composed of

a) 60 to 75 % by weight of an optionally silanized amorphous filling material with a particle size from 1 to 20 µm,

b) 25 to 40 % by weight of an optionally silanized crystalline filling material with a particle size from 0,5 to 1 µm, and

c) optionally up to 10 % by weight of an optionally silanized finely divided silica having an average particle size of less than 0,2 µm.

## Revendications

1. Matière de restauration dentaire, comportant un ou plusieurs composés (méth)acryliques polymérisables, accélérateurs de polymérisation et/ou initiateurs de polymérisation et éventuellement d'autres additifs courants en soi dans des produits de ce genre ainsi que 60 à 90% en poids d'un mélange de charges inorganiques, caractérisée en ce qu'elle contient, comme mélange de charges, un mélange de:

a) 50 à 90% en poids d'au moins une charge amorphe, éventuellement silanisée, et de

b) 10 à 50% en poids d'au moins une charge cristalline, éventuellement silanisée,

chacune présentant un diamètre moyen de particules compris entre environ 0,3 et environ 40 µm, et de

c) éventuellement jusqu'à 10% en poids d'une microcharge présentant un diamètre moyen de particules inférieur à 0,2 µm.

2. Matière de restauration dentaire suivant la revendication 1, caractérisée en ce qu'elle contient, comme mélange de charges, un mélange de 50 à 75% en poids de silicate de lithium et aluminium amorphe, éventuellement silanisé et de 25 à 50% en poids de silicate d'aluminium cristallin éventuellement silanisé, chacun presentant un diamètre moyen de particules compris entre environ 0,3 et 20 µmm, et éventuellement jusqu'à 10% en poids d'un dioxyde de silicium éventuellement silanisé et présentant un diamètre moyen de particules inferieur a 0,1 µm.

3. Matière de restauration dentaire photodurcissable, comportant un ou plusieurs composés (méth)acryliques polymérisables, initiateurs de polymérisation et/ou accélérateurs de polymerisation, éventuellement d'autres additifs courants dans de tels produits et 60 à 90% en poids de la composition totale d'un mélange de charges inorganiques, caractérisée en ce que le mélange de charges est constitué

a) de 60 à 75% en poids d'une charge amorphe, éventuellement silanisée, présentant une dimension de particules comprise entre 1 et 20 µm,

b) de 25 à 40% en poids d'une charge cristalline, éventuellement silanisée, présentant une dimension de particules de 0,5 à 1 µmm, et

c) éventuellement de jusqu'à 10% en poids d'un dioxyde de silicium finement divisé et éventuellement silanisé, présentant une dimension de particules inférieure à 0,2 µm.